# EUROPEAN PATENT APPLICATION

(11) **EP 3 251 617 A1**
(43) Date of publication of application: **06.12.2017**
(21) Application number: 17178084.4
(22) Date of filing: 14.11.2013
(51) Int. Cl.: A61B 17/16, A61C 8/00, A61B 5/00

(54) **DRILL AND TAP AND METHOD FOR PREOPERATIVE ASSESSMENT OF BONE QUALITY**

(30) Priority: 19.11.2012 US 201261728172 P
(62) Divisional of application: 13812143.9
(71) Applicant: Sandvik Intellectual Property AB, 811 81 Sandviken (SE)
(72) Inventor: THEORIN, Anders, SE-302 65 HALMSTAD (SE); MERSON, Eleanor, HUDDERSFIELD, HD8 9AU (GB); LONGDEN, Howard, Mosborough, Sheffield S20 5ES (GB); BJÖÖRN, Dorota, SE-431 69 MÖLNDAL (SE); CARLSSON, Lennart, SE-431 38 Mölndal (SE)
(74) Representative: Neilson, Martin Mark

(57) **Abstract**

A twist drill and bone tap each monitor torque while drilling or threading to assess jaw bone quality and a method for accessing bone quality prior to or while tapping into the bone during a dental implantation procedure. The twist drill for assessing bone quality includes a shank having a proximal section and a distal section. A mounting portion is formed in the proximal section and is adapted to connect with a torque monitoring device. A drill bit is connected to the distal section. The drill bit includes a cutting portion having at least one helical flute formed thereon. A drill point is located at an end of the cutting portion of the drill bit having a helix angle of between about 45 to about 55°, wherein a measurable torque is generated that can be assessed as a function of the quality of the bone material being drilled. A tap for assessing bone quality during tapping includes a shank having opposed ends. A mounting portion is formed in one end and is adapted to connect with a torque monitoring device. A cutting portion is disposed on the other end of the shank and has at least one helical thread formed thereon. A medial, non-cutting portion is disposed between the cutting portion and the mounting portion. The medial portion has a diameter less than a diameter of the cutting portion so as to minimize friction forces.

## Description

### TECHNICAL FIELD AND INDUSTRIAL APPLICABILITY

The present disclosure relates to a twist drill and bone tap that monitors torque while drilling or threading to assess jaw bone quality and a method for accessing bone quality prior to or while tapping into the bone during a dental implantation procedure. The present disclosure aims to maximize the initial implant stability and minimize time to a fully functioning prosthesis.

### SUMMARY

In one embodiment, a twist drill for assessing bone quality includes a shank having a proximal section and a distal section. A mounting portion is formed in the proximal section and is adapted to connect with a torque monitoring device. A drill bit is connected to the distal section. The drill bit includes a cutting portion having at least one helical flute formed thereon. A drill point is located at an end of the cutting portion of the drill bit having a helix angle of between about 45 to about 55°, wherein a measurable torque is generated that can be assessed as a function of the quality of the bone material being drilled.

In another embodiment, a method for assessing bone quality from cutting forces while drilling into bone prior to and an implantation procedure. The method includes the steps of providing a drill, the drill including a shank having a proximal section and a distal section and a mounting portion formed in the proximal section. The mounting portion is adapted to connect with a torque monitoring device. A drill bit is connected to the distal section. The drill bit including a cutting portion having at least one helical flute formed thereon and a drill point located at an end of the cutting portion. The drill bit has a helix angle of between about 45 to about 55°, wherein the helix angle causes torque generated by drilling to become a function of the bone material being drilled. The drill is drilled into the bone and the density of the bone is quantified based upon the measured torque to determine the quality of the bone for implantation.

In still another embodiment, a tap for assessing bone quality during tapping includes a shank having opposed ends. A mounting portion is formed in one end and is adapted to connect with a torque monitoring device. A cutting portion is disposed on the other end of the shank and has at least one helical thread formed thereon. A medial, non-cutting portion is disposed between the cutting portion and the mounting portion. The medial portion has a diameter less than a diameter of the cutting portion so as to minimize friction forces.

In yet another embodiment, a method for assessing bone quality from cutting forces while tapping into bone prior to and during an implantation procedure, including the step of providing a tap including a shank having opposed ends. A mounting portion is formed in one end of the shank and is adapted to connect with a torque monitoring device. A cutting portion is disposed on the other end of the shank and has at least one helical thread formed thereon. A medial, non-cutting portion disposed between the cutting portion and the mounting portion. The medial portion has a diameter less than a diameter of the cutting portion so as to minimize friction forces. A first few threads of the cutting portion is inserted into a predrilled pilot bore of the bone. The density of the material is quantified based upon the measured torque to determine the quality of the bone for implantation.

These and other objects, features, aspects, and advantages will become more apparent from the following detailed description of the preferred embodiment relative to the accompanied drawings, in which:

### BRIEF DESCRIPTION OF THE DRAWINGS

Fig. 1 is a cross-sectional, longitudinal view of the twist drill according to the present disclosure.
Fig. 2 is a diagram of a drill driving device having means for detecting and measuring torque of the drill and a unit for displaying such.
Fig. 3 is a graph measuring the increase in torque for a variety of drills.
Fig. 4 is a cross-sectional view of the bone tap according to the present disclosure.
Fig. 5 is an enlarged perspective view of tap of Fig. 4.
Fig. 6 is an enlarged perspective view of the cutting portion of the tap of Fig. 5.
Fig. 7 is an enlarged perspective view of the medial section of the tap of Fig. 5.
Fig. 8 is a cross-sectional view of another embodiment of the bone tap of the present disclosure.
Fig. 9A graphically represents rounds as function of time, during tapping into 1050 kg/m³ (0-30s) and 300 kg/m³ (30-60s).
Fig. 9B graphically represents torque measured during normal tapping and tapping with an applied extra normal force of approximately 40 N.
Fig. 9C graphically represents examples of raw torque data and data with a floating average of 50 points.
Fig. 10A is a graph of four repetitions of tapping into the 1050 kg/m³ material.
Fig. 10B is a graph of the first and one of the last tapping experiments through the 1050 kg/m³ material.
Fig. 11A is a graph of torque measured when tapping through approximately 5 mm thick samples of 1050, 500 and 300 kg/m³ foam materials.
Fig. 11B is a graph of amplified torque data from the 300 and 500 kg/m³ materials of Fig. 11A.
Fig. 11C is a graph illustrating maximum torque as a function of material density of all individual tapping measurements through approximately 5 mm samples.
Fig. 12A is a graph of torque generated when tapping into thick samples of 500 and 300 kg/m³ foam materials, where the tap did not go through the working material.
Fig. 12B is a graph illustrating torque generated while tapping into a thick sample of the material and tapping through the 500 kg/m³ material.
Fig. 12C is a graph illustrating torque generated while tapping into a thick sample of the material and tapping through the 300 kg/m³ material.
Fig. 13A is a graph of torque measured when tapping into the 500 kg/m³ material with regular tapping procedure, bending forces and pressure forces.
Fig. 13B is a graph of M_{z}max torque measured while tapping into the 300 and 500 kg/m³ materials using a regular procedure, with bending, and with pressure.
Fig. 14A is a graph of torque measured while tapping into the 500 kg/m³ material with a 1.5 mm pilot hole and a 1.2 mm pilot hole.
Fig. 14B is a graph of torque measured while tapping into the 300 kg/m³ material with a 1.5, 1.4 and 1.3 mm pilot hole.
Fig. 14C is a graph of torque measured while tapping into the 500 kg/m³ material with a 1.5, 1.4 and 1.3 mm pilot hole.
Fig. 14D is a graph of torque measured while tapping into the 1050 kg/m³ material with a 1.5, 1.4 and 1.3 mm pilot hole.
Fig. 15A is a graph representing torque measured while tapping into a laminated sample of clamped 1050 to 500 kg/m³ materials and just 1050 material.
Fig. 15B is a graph representing torque measured while tapping into a laminated sample of clamped 500 to 1050 kg/m³ materials and just the 500 material.
Fig. 15C is a graph representing torque measured while tapping into a laminated sample of clamped 1050 to 300 kg/m³ materials and just 1050 material.
Fig. 15D is a graph representing torque measured while tapping into a laminated sample of clamped 300 to 1050 kg/m³ materials and just the 300 material.
Fig. 15E is a graph representing torque measured while tapping into a laminated sample of clamped 500 to 300 kg/m³ materials and just the 500 material.
Fig. 15F is a graph representing torque measured while tapping into a laminated sample of clamped 300 to 500 kg/m³ materials and just the 300 material.
Figs. 16A and 16B are graphs measuring torque generated while tapping into rib samples with cortex, spongiosa and cortex.
Fig. 16C is a graph measuring torque generated while tapping into spongiosa.
Fig. 16D is a graph measuring torque generated while tapping into cartilage-like bone.
Fig. 17 is a graph summarizing the max torque measured into and through different materials while tapping.

### DETAILED DESCRIPTION

Bone quality varies drastically between patients and with the position in the mouth. Evaluation of local bone quality is crucial in dental implantology and dictates the choice of implant type and size, implant placement, implantation strategy, i.e., hole size, need for tapping, etc., and postoperative procedures. A number of techniques are available today to evaluate bone quality, but none are simple, objective and robust enough.

The most common technique for planning implantation surgery is X-ray images of a patient's jaw. It is performed on standard equipment available in all clinics and is powerful in determining local jaw anatomy, such as bone shape, position of nerves and marrow spaces. However, this is not a quantitative method for bone quality assessment, since image contrast is not only dependent on bone density, but also on bone volume, wherein the two are not straightforwardly separable. In addition, the presence of the outermost dense bone will mask the underlying, often less dense bone in the radiographic images. Therefore, use of this method for assessment of bone quality is crucially dependent on surgeon experience. [Branemark P.I. et al, "Tissue Integrated Prosthesis:Osseointegration in Clinical Dentistry", 1985, Quintessence Publishing Co., Inc, Chicago, USA].

Bone quality is also judged by a surgeon by feel during drilling. Bone is then classified into a four category qualitative scale developed by Lekholm and Zarb in 1985:
Quality 1: almost the entire jaw is composed of homogenous compact bone.
Quality 2: dense spongy bone surrounded by thick layer of compact bone.
Quality 3: dense spongy bone surrounded by thin layer of compact bone
Quality 4: low density spongy bone surrounded by a thin layer of compact bone. [Branemark 1985].

This method is fast and part of standard implantation procedure, but highly subjective depending on surgeon experience, tool geometry and tool condition. In addition, while differentiation between hard and soft bone is possible, pinpointing intermediate bone types has been shown to be impossible [Al-Nawas B. et al, "Dental Implantation: Ultrasound Transmission Velocity to Evaluate Critical Bone Quality - An animal Model", Ultraschall in Med, 2008; 29:302-307].

Preoperative bone quality quantification can also be done with computerized topographical methods and 3D X-ray [Turkyilmaz I., McGlumphy EA, "Influence of bone density on implant stability parameters and implant success: a retrospective clinical study", BioMedCentral Oral Health, 2008; 8:32]. However, such equipment is expensive and often not easily accessible. In addition, it requires additional patient examinations and thus additional doses of irradiation.

Ultrasonic techniques for evaluation of bone mechanical properties have been used in orthopedics for some time and are now being researched for dental applications. Speed of sound has for example been correlated to the degree of bone mineralization [Al-Nawas, 2008]. However, so far, the equipment is relatively large and translation into bone quality is not straight forward.

Quantification is also possible from biopsies. However, this is not viable as a standard method in a dental office from a practical point of view. [Friberg B, "On bone quality and implant stability measurements", 1999, Doctoral thesis at "Department of Biomaterials/Handicap Research, Institute for Surgical Sciences, Faculty of Medicine and The Branemark Clinic, Faculty of Odontology, Goteborg University, Goteborg, Sweden].

The idea to measure bone quality from cutting forces when tapping in bone was initially described in a scientific article in 1994 by Johansson and Strid. [Johansson P., Strid K-G, "Assessment of Bone Quality From Cutting Resistance During Implant Surgery", The Intl J of Oral & Maxillofacial Implants, 1994; 9:279-288]. It was further investigated in the PhD thesis by Friberg with a somewhat different approach. [Friberg 1999]. Friberg investigated the correlation between bone quality and cutting forces measured during screw implant insertion and related those to implant stability. Implant insertion torque has also been related to bone quality, where cutting forces have had positive correlation to bone (mineral) density and volume [Friberg 1999], [Homolka P et al, "Bone Mineral Density Measurement with Dental Quantitative CT Prior to Dental Implant Placement in Cadaver Mandibles: Pilot Study", Radiology, 2002; 224:247-252]. Where higher bone density has been correlated with a higher implant success rate. These studies have resulted in a number of commercial instruments to monitor implant insertion torque during operation, for example Osseocare^{®} manufactured by W&M of Windsor, ON and available from Nobel Biocare AB of Gothenberg, SE. However, such instruments are not used pre-operatively.

Thus, the original approach of extracting bone quality from a tapping procedure during implant site preparation was replaced by analyzing forces during the final operational step of implant insertion. A large shortcoming of this approach is that information on bone quality is available only after implant placement.

Typically, quantification of implant stability is often done after the implant placement. The available technique uses resonance frequency. Unfortunately, it is only useful in the post-operative treatment planning, and not during surgery planning, which is crucial for optimization of initial implant stability. Commercial instruments are marketed by Osstell (osstell.com of Gothenberg, Sweden), and are available from many dental companies.

Another shortcoming in the field identified by the Johansson study is the tap design itself, where friction from the threads and chip packing during tapping procedure contributed largely to the true cutting forces. [Johansson 1994]. This approach required complicated data analysis and a number of assumptions. In addition, the regular dental tap used in this study has advances slowly with approximately 0.6 mm/turn.

Thus, an objective, quantitative method for jaw bone quality assessment prior to implantation is needed. The approach of the present disclosure is to measure bone quality from cutting forces when tapping into bone during the implantation procedure. Such approach enables simple and objective bone quality quantification as part of the operational procedure, which in turn enables procedure optimization in the operation room. Thus, the present approach is inexpensive, fast and can become a standard method in all operating rooms. It should be appreciated that bone quality in a variety of different bones can be assessed according to the present disclosure.

The field of dental drilling is well developed, with several patents already existing in this area. However, the key issue addressed by other inventions (e.g. U. S. Patent Nos. 5,569,035 and 5,762,498) is that of overheating of the bone during drilling, and potential fluting of the holes. Most dental drill patents existing in the prior art focus on geometry of coolant channels and/or flute design, because of the temperature control issue.

However, the aim of twist drill of the present disclosure is not to minimize the heat formed, although by using a helix angle higher than those used generally it is expected that the heat generated by the present drill will not be excessive, but rather to provide a drill that assesses the measurement of torque independent of the user, to give an indication of the quality of the bone.

Accordingly, the twist drill of the present disclosure has a constant flute without splitting on the point, to maximize the length of the chisel, and therefore to reduce the pull through of the drill should it hit a cavity.

The twist drill of the present disclosure is also designed to be used in applications where further drilling and/or tapping operations are necessary and in conjunction with equipment to measure accurately the torque while drilling. As well as such that measurement of torque may be assessed, independent of user, to give an indication of the quality of bone.

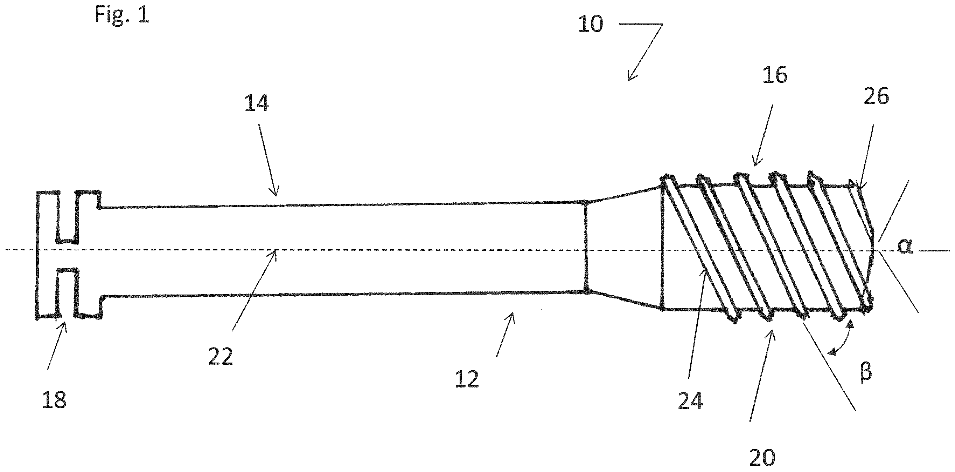

Referring to Fig. 1, a twist drill 10 of a disclosed embodiment is adapted for assessment of jaw bone quality by torque monitoring. Drill 10 includes a shank 12 having a proximal section 14 and a distal section 16. Proximal section 14 includes a mounting portion 18. As is known, and which will be described further herein, mounting portion 18 has any suitable configuration to enable the drill to connect with a driver and equipment to accurately measure torque during the drilling process. Distal section 16 includes a drill bit 20 located at an end thereof. Drill bit 20 is designed to be used in applications where further drilling and/or tapping operations are necessary.

Drill 10 rotates about a longitudinal, rotary axis 22. Shank 12 can be a single piece of steel, for example, Sandvik Bioline 4C27A or any other stainless steel suitable for medical devices. However, it should be appreciated that other materials and configurations of drill 10 are contemplated by the disclosed embodiment. Moreover, the drill could be coated for the purpose of wear detection and better visualization during operation. There could also be markings on the tools indicating, for example, how deep the tool is inserted into the bone.

Drill bit 20 includes at least one continuous helical flute 24 that winds around distal section 16. Although a single flute is illustrated it should be appreciated that two or more flutes can be provided. A drill point or bit 26 defines a cutting edge, which cuts into the bone and tissue. Drill bit 26 has no splitting on the point to maximize the length of the chisel. Thus, the pull through of the high helix drill is reduced, especially should it hit a cavity.

As will be described further herein, the disclosed embodiment demonstrates that as the helix angle of the drill increases, the variation in torque with varying feed drops. Thus, with a drill having a large helix angle, the torque generated becomes a function of the properties of the material being drilled, and does not vary as much depending on the force applied during drilling or the skill of the surgeon.

Referring again to Fig. 1, flute 24 of the disclosed embodiment has a helix or spiral angle β in the range of about 45° to about 55 °, and preferably about 50 °. Also as shown in Fig. 1, the twist drill bit 20 has a point angle α of or between about 125° to about 135 °, most preferably about 130 °.

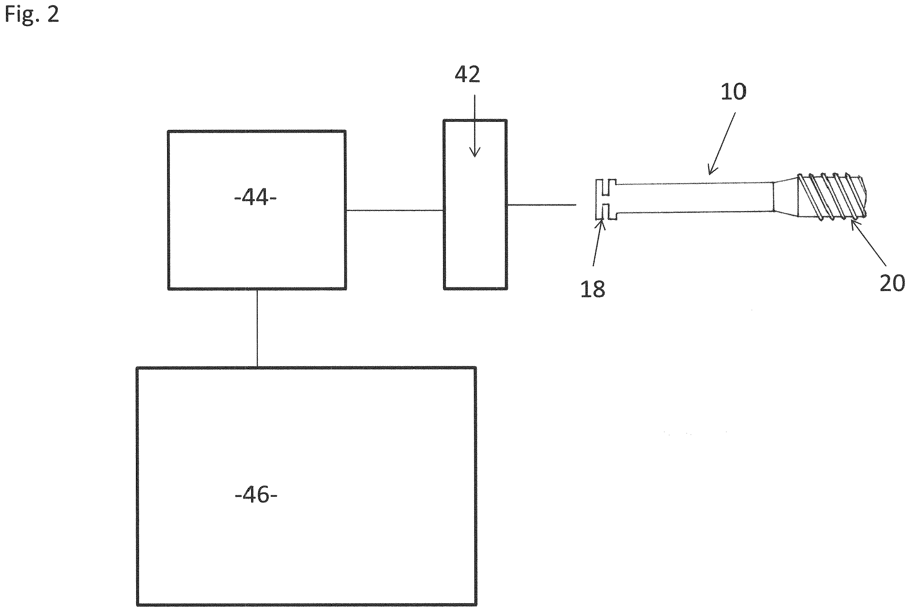

Referring to Fig. 2, the drill of the disclosed embodiment is adapted to be connected to a known device/means for monitoring torque. The mounting portion 18 of drill 10 can be connected with a torque transferring mechanism 42, such as a hand piece or dental driver. Mechanism 42 is connected with torque detector means 44, which measures torque with known means. A display unit 46 displays a torque curve that is monitored to access the quality of the bone tissue. Such torque detection and display means are commercially available, for example, Osseocare® manufactured by W&M of Windsor, ON and available from Nobel Biocare AB of Gothenberg, SE. See also U.S. Patent No. 6,951,698.

Simulations of drilling were carried out to find the parameters that had the largest influence on variation of torque with change in feed. The results showed conclusively that as the helix angle increases, the variation in torque with varying feed drops. Therefore, by using a drill with a large helix angle, the torque generated becomes a function of the properties of the material being drilled, and does not vary as much depending on the force applied during drilling. However, helix angles of much more than about 50° make it difficult to produce a straight cutting edge. A high helix angle of, for example, about 45°, was chosen to achieve a tap requiring minimum of normal force for efficient tapping. A further advantage of a high helix angle is that it gives an extremely sharp cutting edge, which reduces cutting forces over-all, and also minimizes the heat generated.

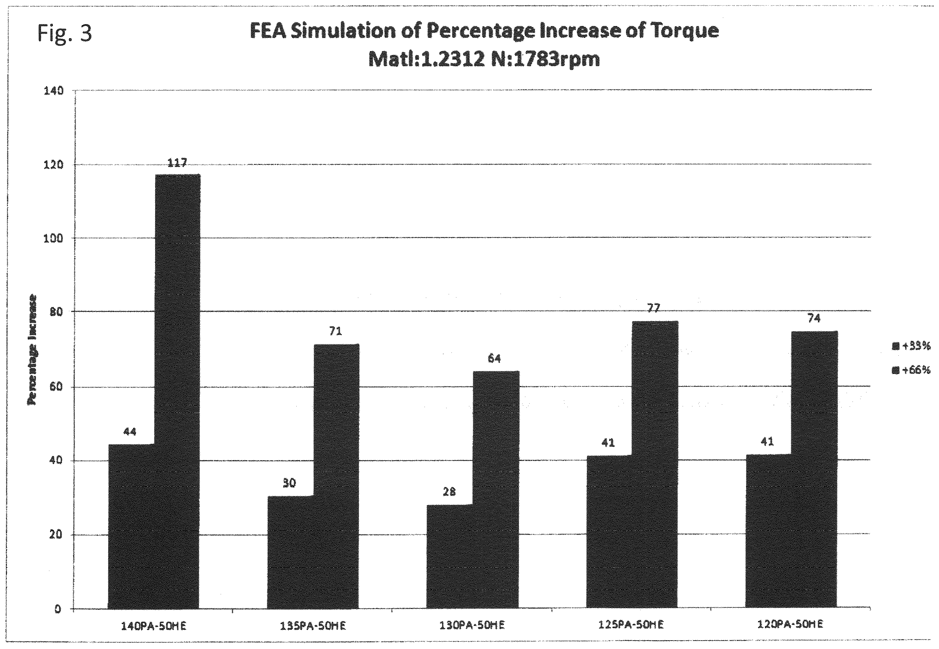

A similar simulation was later carried out with variation in the point angle with different helix angles. The results of this simulation are shown in Figure 3. The experiment was carried out at three different feed rates - a minimum, minimum +33%, and minimum +66%. These feed rates represent the speeds that different dentists using the tool in hand drilling applications may apply. Speed was maintained at a constant level. The results show that a point angle of about 130° gave the least increase in torque as the feed was increased. Because clearance angles are not expected to have an influence on torque, standard primary clearance angles are used. The clearance face will not come into contact with the bone during the drilling operation. As long as clearance is sufficient to avoid contact of parts of the point other than the cutting edge during drilling, values may vary. Table 1 below summarizes the point angle-helix angle combinations tested to determine optimum helix angle, per the graph of Fig. 3 showing the results.

**Table 1**

| **Tool ID** | **Point Angle** | **Helix Angle** |
|---|---|---|
| 120-50L | 120° | 50° |
| 125-50L | 125° | |
| 130-50L | 130° | |
| 135-50L | 135° | |
| 140-50L | 140° | |

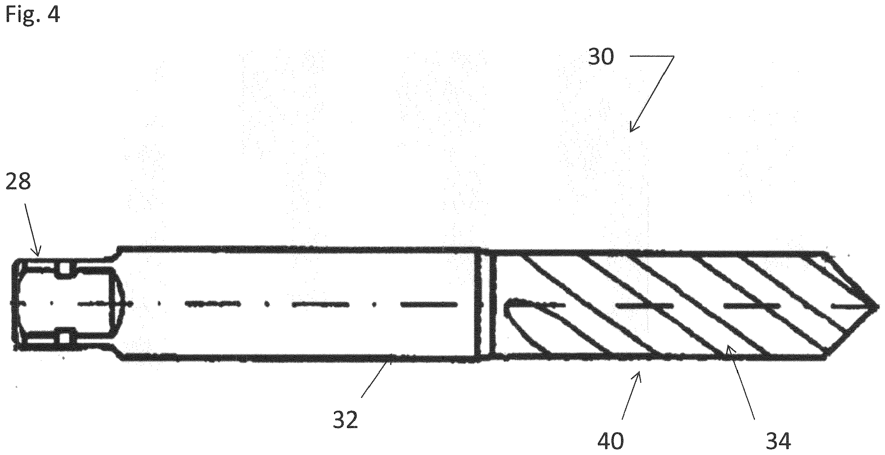

The present disclosure also encompasses a dental tap for the assessment of bone quality. Referring to Figs 4-7, the tap design of the disclosed embodiment minimizes friction forces and to enable faster advancement into a hole in the patient's bone and tissue compared to conventional dental taps. As shown in Fig. 4, a tap 30 includes a shank 32 having opposed ends. A first end includes a mounting portion 28 and a threaded tap section 40 disposed at a second, opposed end. Tap section 40 includes at least one thread 34, which will be described further herein.

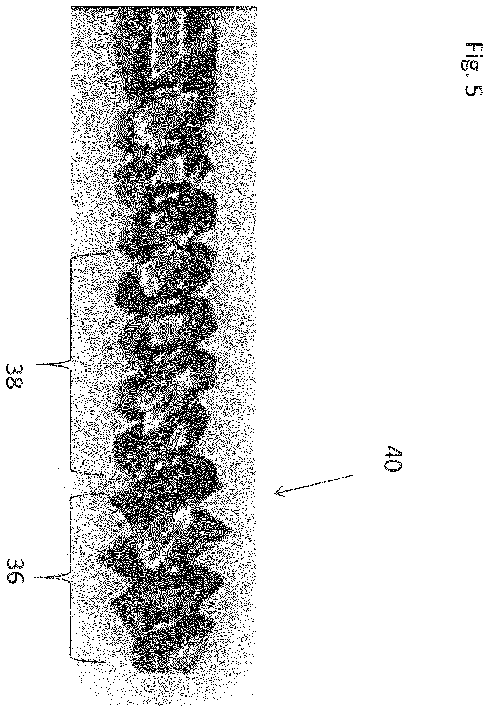

As shown in Figs 5-7, tap section 40 includes a cutting portion 36 followed by a medial, non-cutting portion 38. For boring a standard pilot hole of 1.5 mm, a tapping length of the drill can be approximately about 10 mm with a thread height of about 1 mm/round. The distance between the threads can be about 1 mm, thus, the tap advancing about 1 mm with each turn. It should be appreciated that the dimensions of the tap depend upon the requirements of the tap hole or bore. Accordingly, the tap of the present disclosure is not limited to a specific length, diameter or thread height, etc.

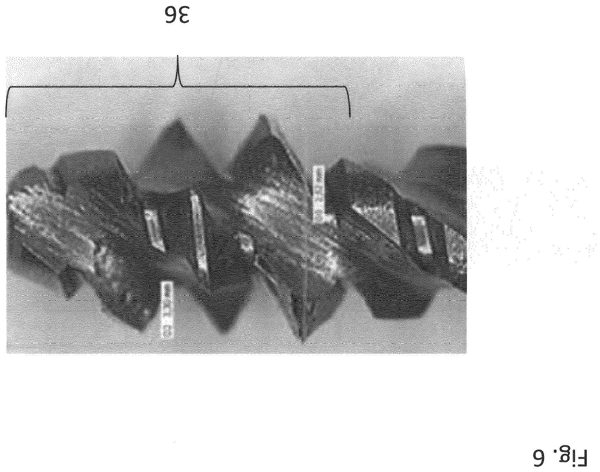

Fig. 6 is an enlarged view of cutting portion 36. As shown, cutting portion 36 has a length of approximately the first four threads. In practice, the approximate first three threads being tapered can guide the tap into a predrilled hole of, for example, about 1.5 mm. The 1.5 mm diameter corresponds to a tap width at approximately half the first thread, so about 0.5 mm of tap could be inserted into the hole prior to threading. The forth thread can be full, having a diameter of about 2.5 mm and at a distance of approximately about 3.3 mm from the tip. These dimensions too are only exemplary and not intended to be limiting. For example, 80 % of all dental implants placed have a diameter of about 3 to about 4.5 mm which implies a final drill diameter of about 2.5 to about 4 mm. Total range is about 2.5 to about 6.5 mm. Also, thread height is about 0.4 to about 0.6 mm for most implants. Approximately 90 % of all implants have a length of about 7.5 to about 18 mm, the remainder being shorter with a larger diameter. This corresponds to the same drill length plus approximately one mm for the cutting portion. A shaft length of some cm plus connection is needed for visibility when working.

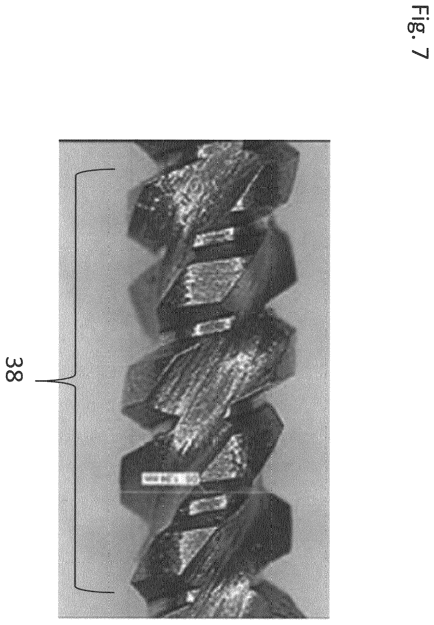

Referring to Fig. 7, medial portion 38 can be comprised of about the remaining 6 threads with a diameter that is reduced to approximately 50% to minimize the friction contribution, thereof resulting in, for example, a diameter of approximately 2 mm. Once again, it should be appreciated that these dimensions and lengths are exemplary only and not intended to limit the present disclosure.

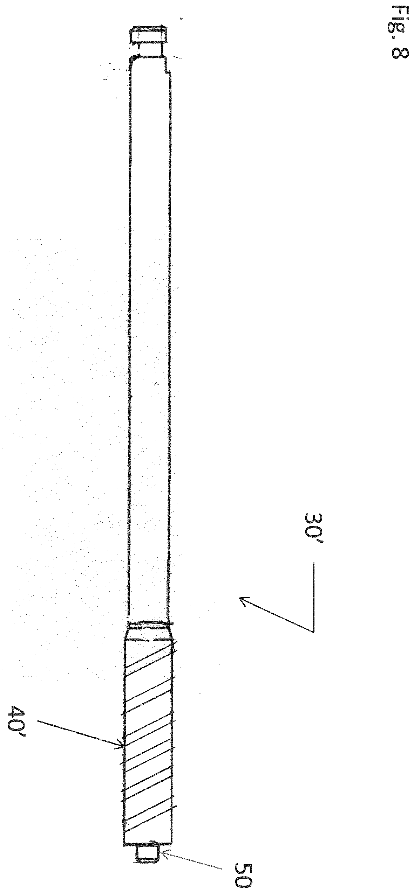

In another embodiment shown in Fig. 8, the tap 30' can include a non-cutting, steering portion 50 located on the end of the tap section 40'.The steering portion of the tap is used for guidance of the tool into the predrilled hole minimizing or removing the risk of tapping outside of the pilot hole direction
All threads, angles, etc. are the same in both embodiments of the tap.

The present disclosure further provides a method for assessing bone quality from cutting forces while tapping into bone prior to and during an implantation procedure. The first few threads of cutting portion 36 are inserted into a predrilled pilot hole (not shown) of the bone. The tap is incrementally tapped into the hole and the torque produced by each turn is monitored by a detecting means, for example, similar to means 44 shown in the embodiment of Fig. 2. During this tapping, cutting portion 36 is advanced of about 1mm with each turn. The measured torque is displayed by unit 46 and the user can quantify the density of the material based upon the measured torque to determine the quality of the bone by comparing the measured torque to predetermined torque values of classified bone qualities.

Experiments were performed using the tap of the disclosed embodiment, which was evaluated as a tool for preoperative assessment of jaw bone quality. The approach was based on torque monitoring while threading in foam of various densities and in bone. It was determined that the present tap design minimized friction forces from non-cutting part of the tool and was suitable for assessment of cutting torque while threading. Further, materials of densities relevant for bone could be distinguished by torque measurements, with sufficient sensitivity and reproducibility. Also, the achieved depth resolution was about 2 to about 3 zones per 10 mm tapping, which was relevant for dental implantology application. It was further shown that tap performance was relatively insensitive to application of bending and normal forces.

The tap of the present disclosed embodiment has been designed to minimize friction forces and to enable faster advancement into the hole. A high helix or spiral angle of about 45° was chosen to achieve a tap requiring minimum of normal force for efficient tapping. Tapping length was approximately about 10 mm with thread height of about 1 mm/round into a standard pilot hole of about 1.5 mm. The cutting portion of the tap consisted of approximately the first 4 threads. About the first 3 threads guided the tap into the predrilled pilot hole. The 4th thread was full with a diameter of about 2.5 mm. The remaining approximate 6 threads were reduced to approximately 50% to minimize friction contribution, resulting in a diameter of approximately 2 mm.

In the tests, polyurethane foam with densities of about 300 kg/m3, about 500 kg/m3 and about 1050 kg/m3 (from Technipur AB of Västervik, SE) as well as bovine ribs, (herein referred to as "300," "500," "1050" and "bone") were used as the working experimental materials. The performed tapping tests were: i) through about 5 mm thick slices of foam material; ii) into greater than 10 mm thick pieces of foam material; iii) into/through laminated samples consisting of two approximately 5 mm foam slices of different density clamped together; and iv) into bone.

All tests were done manually using a hand piece and torque (M_{z}) and rounds were monitored as a function of time. Torque was measured by a plate, which the material was clamped onto and the centrum of the torque plate was indicated by a laser beam. Each tapping experiment was repeated about 3 to 4 times.

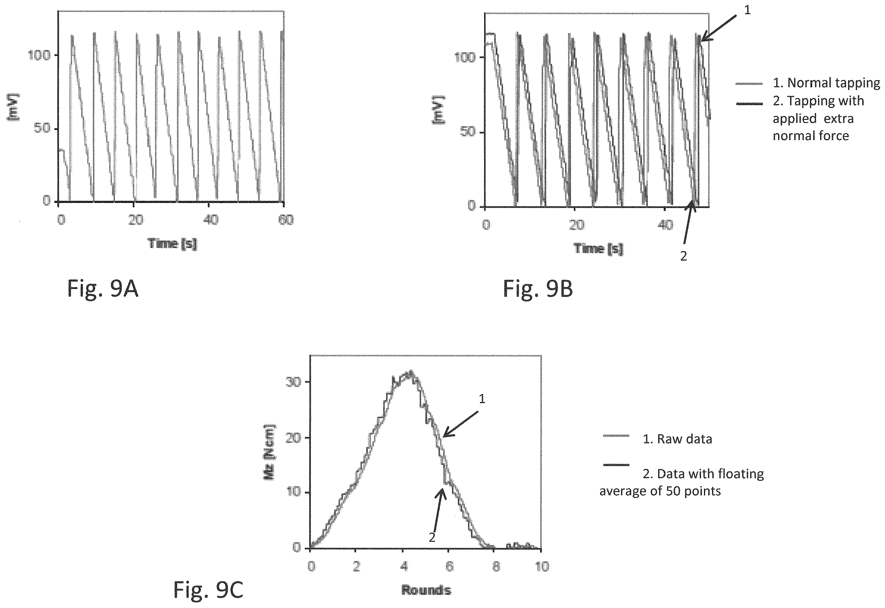

Referring to Figs. 9A-C, data for both torque and rounds was sampled every approximately 20 ms in mV, where each mV for M_{z} corresponded to about 0.035 Ncm. Each measurement is presented separately in the figures, since mean values are not relevant for the bone quality evaluation application, where in practice only one measurement can be performed per site. Tapping speed was approximately 10.6 rpm or approximately 22.8 rpm and as shown in the graphs was not affected by material density (as shown in Fig. 9A), applied forces (as shown in Fig. 9B) or tapping depth (Figs. 9A and 9B). For consistent presentation all data was potted with M_{z} as a function of the number of rounds with data floating an average of about 50 points (see Fig. 9C) and the base line adjusted to zero.

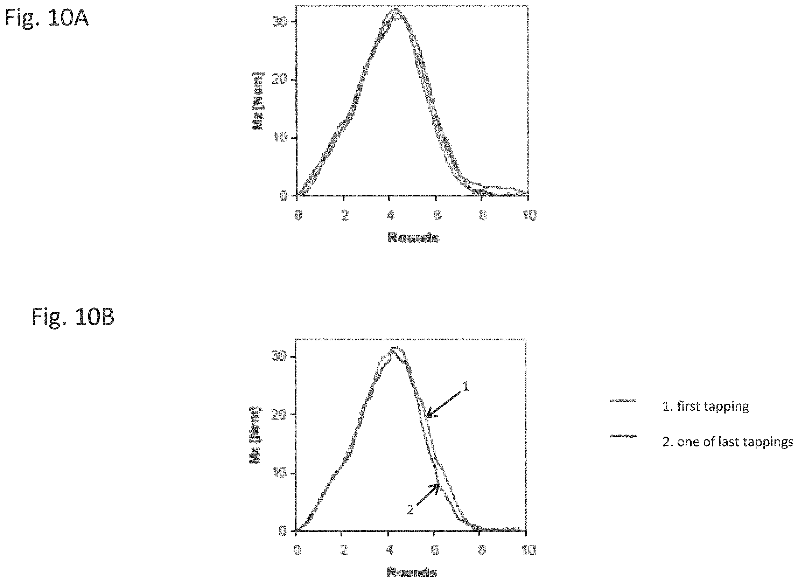

As stated previously, all tapping experiments were repeated about 3 to 4 times under the same conditions with excellent reproducibility. All data presented was acquired using a single tap, the performance of which did not appear to change over the course of the experimental series. Fig. 10A shows an example of tapping through the 1050 material several times, with each curve representing a tapping. Fig. 10B shows that the first and one of the last tapping experiments resulted in very similar M_{z} profiles. In addition, the first experiment was performed at double rpm compared to the last. The similarity between curves indicated that approximately 10 rpm or approximately 20 rpm did not influence the M_{z} responses.

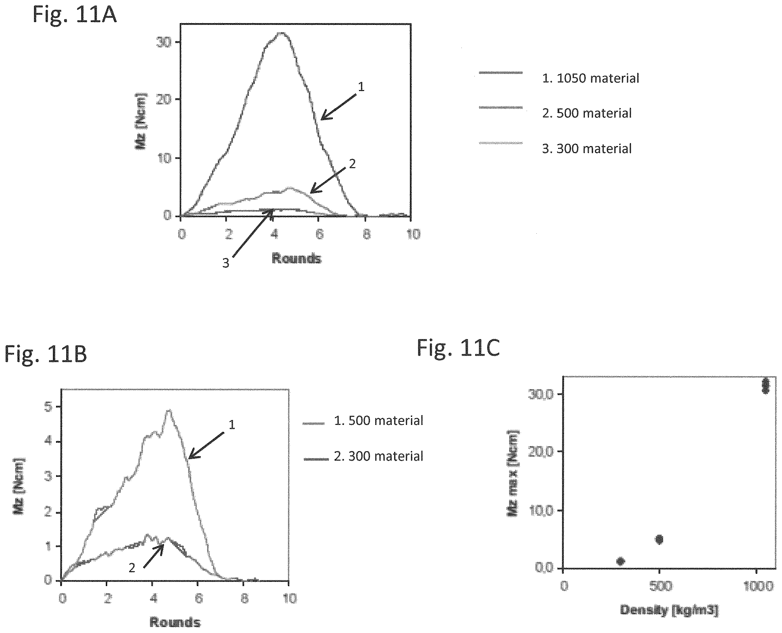

Figs. 11A-11C show that maximum tapping torque increases with material density. The graph of Fig. 11A illustrates examples of torque curves for tapping through approximately 5 mm pieces of 1050, 500 and 300 foam, with Fig. 11B presenting amplified data of the 300 and 500 materials of Fig. 11A. As shown, the torque (M_{z}) increased approximately linearly during the first approximate 4 to 5 turns, where a maximum torque was reached. This corresponded to about the top 5 threads, thus approximately 5 mm of the tap, placed in the material. As soon as the tap entered the exit hole, the torque decreased and reached 0 at approximately 8 rounds thus at approximately 8 mm irrespective of the foam type. This should correspond to the situation where the full thread, located approximately 3.5 mm from tip, exited the approximately 5 mm thick material. In addition, the results indicated that the friction contribution to the torque M_{z} from the upper part of the tap was minimal. Comparison of M_{z}max from tapping through 1050, 500 and 300 foams showed low spread between comparable measurements and large differences between material types as shown in Fig. 11C.

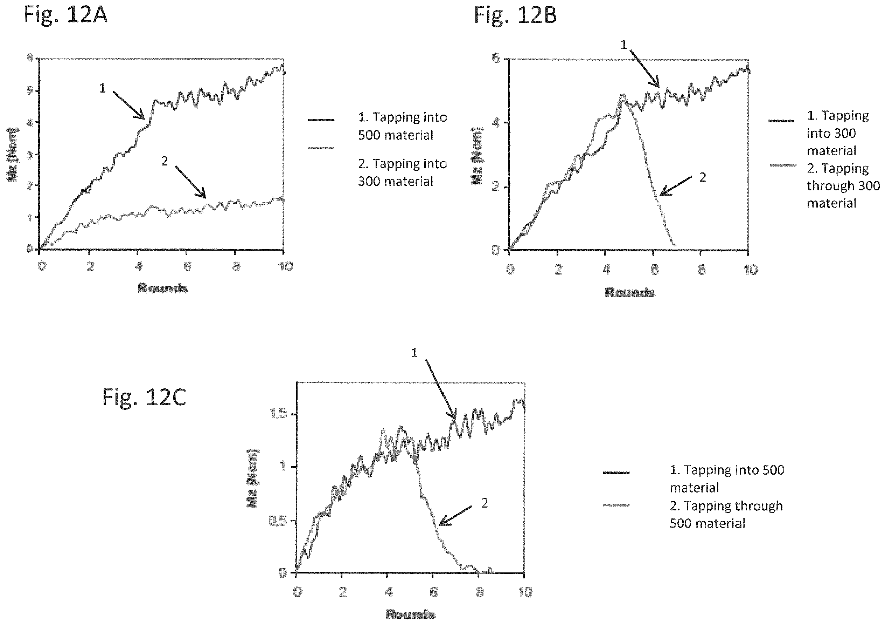

Referring to Figs. 12A-12C, torque experiments tapping deep holes into samples thicker than the tapping length showed similar trends comparable to tapping through the materials. As shown in Fig. 12A, torque was affected by material density with initial M_{z} behavior comparable to tapping through experiments of Figs. 12B and 12C for the 500 and 300 materials, respectively. Torque increased approximately linearly up to approximately 4.5 turns, approaching a constant value with the increasing number of tapped threads. A plateau was expected for the rest of the process if torque was the only force acting on the tap. However no such plateau was reached. Instead a slow increase in M_{z} was observed, see Fig 12A (deep hole tapping for rounds > 4.5). Since contribution from upper threads has been considered to be minimal, the continuous increase of M_{z} in deep holes might instead have been caused by chip packing around the tap. Such trend was in agreement with previous observations and interpretations (see Johansson, 1994).

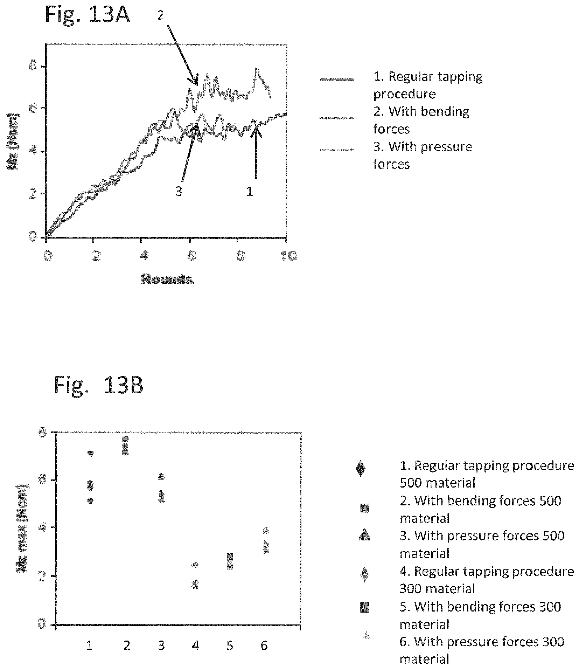

Experiments were also conducted into the influence of applied forces and pilot hole diameter on torque produced by the tap of the disclosed embodiment. Stability of the tap performance was evaluated by applying bending or normal forces while threading. Provocation by bending the tap approximately ±15° or pressing with approximately 40 N with frequency of approximately 0.5 Hz had some influence on torque compared to regular tapping. Fig. 13A shows examples of provocation in the 500 material, where regular threading was used up to approximately 3.5 rounds, after which bending or pressure was applied. Fig. 13B is a summary of torque M_{z} max from all measurements with and without provocation. Even though some influence on torque is observed, it should be pointed out that the magnitude of provocation used here was much higher than applicable in dental practice. Thus, torque is relatively insensitive to bending and pressure forces.

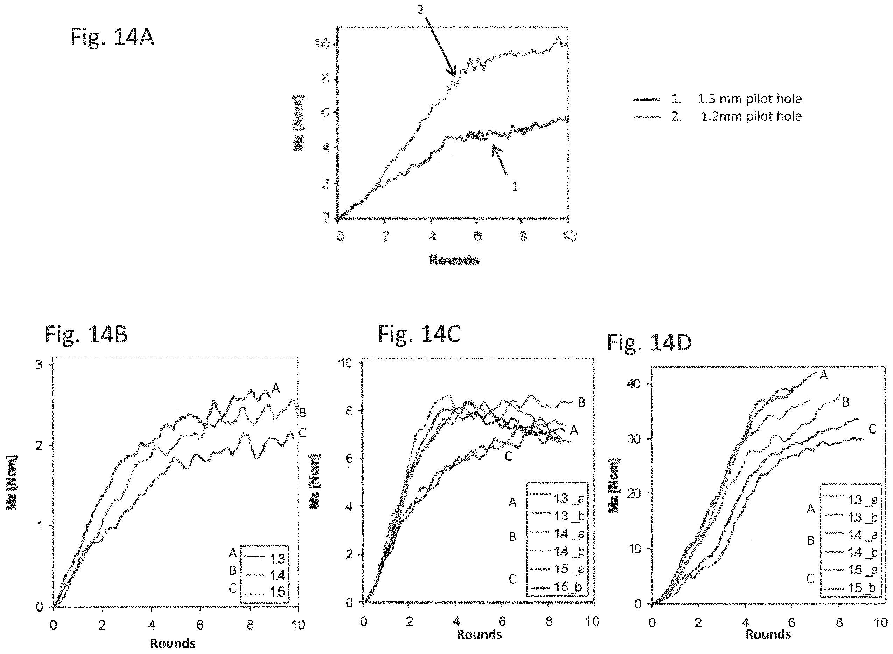

The tapping procedure was performed in polyurethane foam (from Technipur AB of Västervik, SE) having densities of approximately 300 kg/m³, 500 kg/m³ and 1050 kg/m³ after pre-drilling pilot holes of about 1.2, 1.3 mm, 1.4 mm or 1.5 mm diameters. Results of the tests are shown in Fig. 14A-D. Measurements were performed in duplicate, but are presented separately since mean values are not relevant for the bone quality evaluation application, wherein practice only one measurement can be performed per site. Data for both torque and number of rounds was sampled in mV, where each mV corresponded to M_{z} of 0.1005 Ncm with maximum equipment limit in M_{z} of approximately 40 Ncm. Tapping speed was approximately 22.7 rpm. For consistent presentation, all data was potted with M_{z} as function of number of rounds with data floating average of about 50 points and base line adjusted to zero.

Changes in M_{z} as a function of tapping depth for three material densities and three pilot hole diameters are shown in Figs. 14B-D. Reproducibility at each condition (material density and pilot hole diameter) was satisfactory. Fig. 14B shows that for the 300 kg/m3 material M_{z}, max increased by a factor about 1.24 for a 1.3 mm pilot hole and about 1.16 for a 1.4 mm pilot hole compared to the 1.5 mm hole diameter. For the 500 kg/m3 material, shown in Fig. 14C, M_{z}max increased by a factor of about 1.15 for the 1.3 mm pilot hole and about 1.16 for the 1.4 mm pilot hole compared to the 1.5 mm diameter hole. For the 1050 kg/m3 material, M_{z} could not be evaluated to the full depth due to equipment limit at approximately 40 Ncm, see Fig. 14D. A comparison of the six rounds showed that M_{z} increased by a factor of about 1.39 for the 1.3 mm pilot hole and about 1.18 for the 1.4 mm hole compared to the 1.5 mm diameter hole. This 1.39 factor was the largest increase noted and might not be representative considering that M_{z},max was not yet reached. As expected, the increase in M_{z} was inversely proportional to pilot hole diameter. It can be seen that the differences in torque due to material density (comparison of Figs. 14B-14D) were much larger than variations due to pilot hole diameter for each density.

Experiments tapping through laminated materials were conducted with the tap of the disclosed embodiment and consisted of tapping into/through working materials consisting of two clamped together approximately 5 mm pieces of various foam densities. For comparison, an approximately 5 mm thickness of working material of the same density as the first piece in the laminated samples was tapped through.

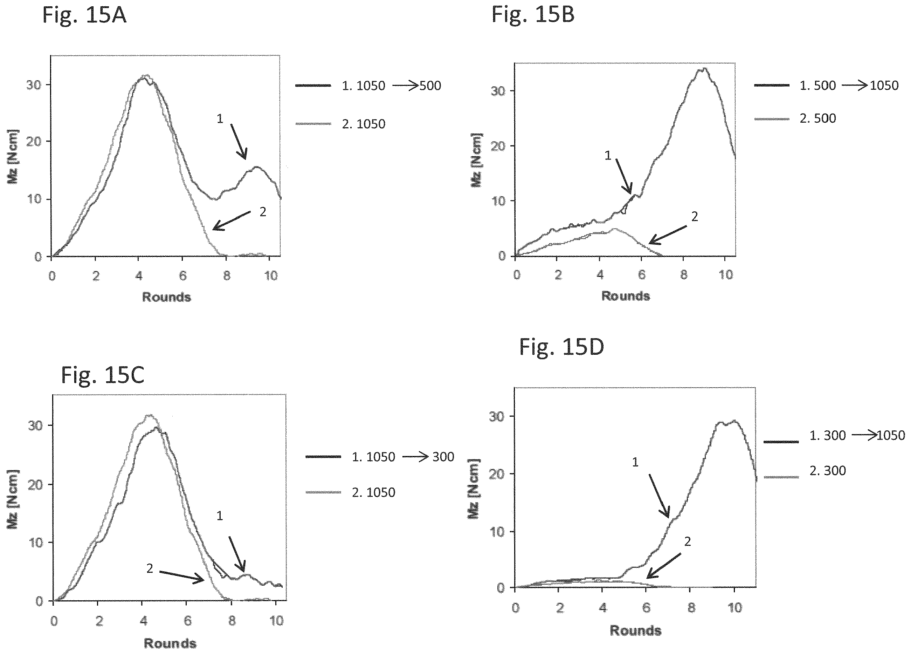

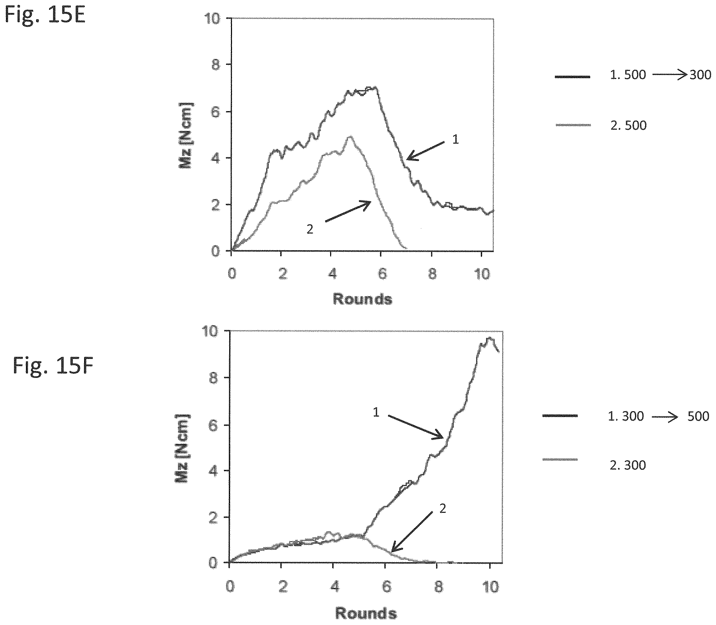

Figs. 15A-15F show examples of tapping through experiments, where one curve represents torque measured tapping through the clamped, laminated materials and the other curve tapping through just the top layer. In the experiment of Fig. 15A, about a 4.5mm layer of 1050 kg/m³ material was clamped to about a 4.6 mm layer of 500 kg/m³ material, with tapping occurring from the 1050 material to the 500 material. The remaining graphs illustrate tappings as follows: Fig. 15B - 500 (4.6mm) to 1050(4.5mm); Fig. 15C - 1050 (5.0mm) to 300 (3.2mm); Fig. 15D - 300 (3.2mm) to 1050 (5.0mm); Fig. 15E - 500 (5.4mm) to 300 (5.3mm); and Fig. 15F - 300 (5.3mm) to 500 (5.4mm). Results showed that the tap could recognize the presence of two material types. In a typical curve, tapping through the first layer showed similar behavior as in tapping through experiments, with a maximum in M_{z} after about 4 to 5 rounds and M_{z} approaching 0 Ncm at approximately 8 rounds. Thus, at 8 rounds and further into the material the M_{z} response should originate mainly from the second material layer. Tapping into the second less dense layer resulted in a decrease in torque at higher rounds (Fig. 15A, 15C and 15E) until a stable level was reached at approximately 8 rounds (Fig. 15C and 15E). The opposite was true for crossing from less to more dense materials, where torque increased instead (Figs. 15B, 15D and 15F). The sudden dip in M_{z} towards the end of tapping, observed in most curves, corresponded well with the tap reaching the exit hole of the laminated samples.

Tapping into laminated samples in most cases resulted in higher torque compared to simple tapping through or into deep holes. This trend could be observed for both the first (for example, Fig. 15B) and the second (for example, Fig. 15A) material layers. For example, in the latter (Fig. 15A) a M_{z} max of approximately 14 Ncm was measured for the 500 portion of the laminated sample, while approximately 5 Ncm was typical for tapping through and approximately 6 Ncm for deep holes of the same material density. Another observation was around the dip in M_{z} when passing from the 1050 to 500 kg/m³ material (Fig. 15A). A plateau behavior was expected instead of the observed top at approximately 9 to 10 rounds, probably due to the experimental setup with possible chip packing and air pockets between materials or stresses from clamping. Elasticity of the material also could have influenced M_{z}.

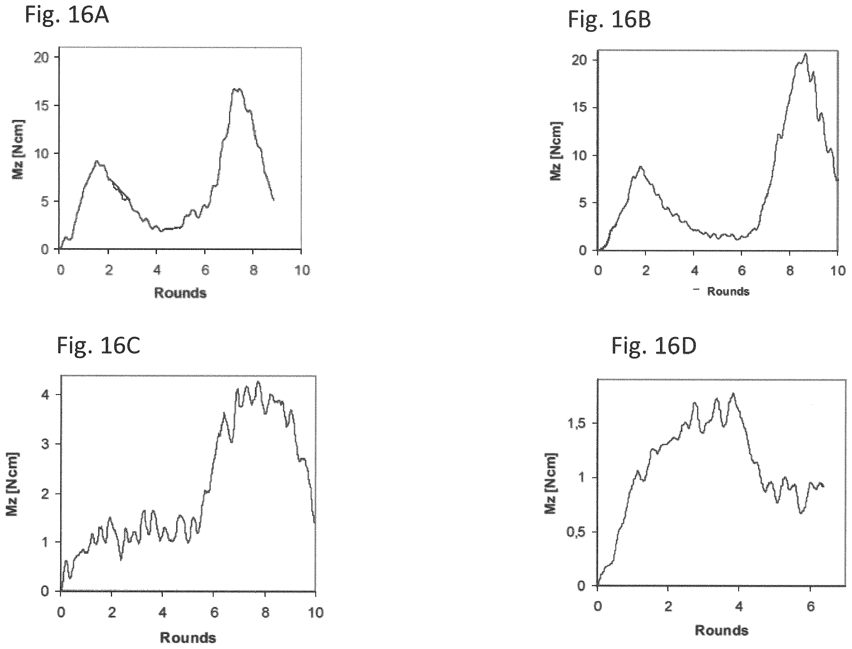

Torque was also measured in various types of bone using the tap of the present disclosure. Referring to Figs. 16A-16D, tapping in bone showed that a distinction between several bone types was possible, both within the same sample and between sample types. Bone density was not quantified in the specific specimens used, instead several qualitatively different working materials were chosen. For example, tapping into a flat rib showed two peaks separated by a zone of low torque (Figs. 16A and 16B) corresponding to passing through cortex, spongiosa and cortex. The sudden drop in torque M_{z} towards the end of the measurements was explained by tapping through the working material. Tapping through closely spaced holes in the same piece of bone was also qualitatively reproducible (Figs. 16A and 16B). Quantitative comparison was not attempted considering bone being a highly non-homogenous material.

Tapping was also performed in softer bone qualities. For example, as shown in Fig. 16C a spongiosa sample appears to consist of one less and one more dense layer. As shown in Fig. 16D, a "cartilage-like" bone resulted in very low torque values, where after an initial increase in M_{z}, torque decreases possibly due to a cavity or air pocket. It should be appreciated that the data interpretation can only be considered as speculative, as the specific bone specimens were not analyzed by other techniques.

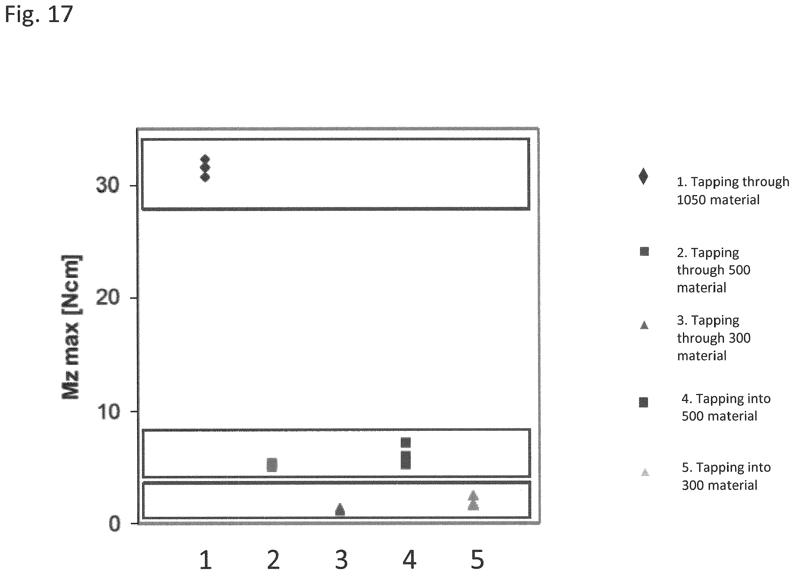

As discussed supra, the screw tap of the disclosed embodiment was designed and evaluated as a tool for assessment of jaw bone quality during hole preparation sequence of the operation. The approach was based on torque monitoring while threading in foam of various densities, and in bone. The tap was designed with approximately 3 threads leading the tool into a pilot hole, with one full thread and the remaining threads being considerably reduced in diameter. With such design, friction forces from the non-cutting portion of the tap were minimized and materials of different densities could be distinguished. Thus torque could be used for quantification of material density. Examples of M_{z} max for tapping through or into 1050, 500 and 300 kg/m³ materials are summarized in the graph of Fig. 17, wherein rectangles frame data of each material density.

In a clinical application, the goal should be to distinguish four classes of bone, where approximately 800 kg/m³ is typical for cortical bone and ∼300-500 kg/m³ for soft bone. Thus, the sensitivity of M_{z} in the current experiments was sufficient. However, to be able to relate current data from foam materials to bone, more tapping tests in bone together with bone anatomy quantification are necessary. Tapping should also be performed in living tissue, where forces will be affected by presence of blood and fat. This natural lubrication will have to be considered constant.

Depth resolution of the tap of the disclosed embodiment showed clearly 2-3 zones, which were sufficient for dental implantology application. A total evaluation depth of about 10 mm was relevant for the dental application considering that approximately 70% of implants used today are 10-12 mm long.

Tap performance was relatively insensitive to application of bending and normal forces, but somewhat affected by the size of the pilot hole. The latter may pose a problem in dental practice, where quality of pilot hole (size and shape) might be difficult to control. One solution would be to always recommend a smaller pilot diameter then intended for the tool in combination with a somewhat self-advancing tap. However, the tool should not be more aggressive so as to not easily follow the path guided by the pilot hole.

M_{z}max was used in the above experiments as a distinction factor, however, another optimal approach to data analysis could be appreciated. For example, torque could be related to bone hardness and elasticity rather than density. Possibly bone elasticity could be deduced by monitoring tap retraction forces. Finally, data from several taps should be compared.

It should be appreciated that the tap of the present disclosure contemplates that in addition to assessing material quality from tapping torque forces a drill capable of sensing material quality has also been developed. Both tools can simply be included into the existing dental implantology tool kits.

Furthermore, the twist drill and tap of the present disclosure both could be provided, wherein a tap could be an addition to the procedure, while a drill could replace an already existing tool in a set, which would be preferred. Possibly a combination of the two would be the most attractive. In such solution, the first drilling step of typically 1.5 mm-2.0 mm would be exploited for the first indication of bone quality. A tap could then be available, if more rigorous analysis was needed.

The tap, drill and methodology of the present disclosure can also function with software developed for analysis and visualization of the cutting data. It should be further appreciated that the tap, drill and methodology of the present disclosure can be useful in other areas, such as orthopedics.

Although the present disclosure has been described in relation to particular embodiments thereof, many other variations and modifications and other uses will become apparent to those skilled in the art. It is preferred therefore, that the present disclosure be limited not by the specific disclosure herein, but only by the appended claims.

### ITEMIZED LIST OF EMBODIMENTS

1. A twist drill for assessing bone quality, comprising:
   a shank having a proximal section and a distal section;
   a mounting portion formed in said proximal section, said mounting portion being adapted to connect with a torque monitoring device;
   a drill bit connected to said distal section, said drill bit including a cutting portion having at least one helical flute formed thereon; and
   cutting means disposed at an end of said cutting portion of said drill bit for cutting bone material, wherein said cutting means generate a measurable torque that can be assessed as a function of the quality of the bone material being drilled.
2. The twist drill according to item 1, characterized in that the cutting means comprises a drill bit point having a helix angle of about 45° to about 55°.
3. The twist drill according to item 1 or 2, characterized in that the drill bit point has a helix angle of about 50°.
4. The twist drill according to any of the preceding items, characterized in that the drill point has a point angle of about 125 ° to about 135°.
5. The twist drill according to any of the preceding items, characterized in that the drill point has a point angle of about 130°.
6. The twist drill according to any of the preceding items, characterized in that the drill bit has a plurality of flutes formed thereon.
7. A method for assessing bone quality from cutting forces while drilling into bone prior to and an implantation procedure, comprising the steps of:
   providing a drill, said drill including a shank having a proximal section and a distal section, a mounting portion formed in said proximal section, said mounting portion being adapted to connect with a torque monitoring device and a drill bit connected to said distal section, said drill bit including a cutting portion having at least one helical flute formed thereon, and a drill point located at an end of said cutting portion of said drill bit, said drill bit having a helix angle of about 45 ° to about 55°, wherein said helix angle causes torque generated by drilling to become a function of the bone material being drilled;
   drilling into the bone; and
   quantifying the density of the bone based upon the measured torque to determine the quality of the bone for implantation.
8. The method according to item 7, characterized in that the drill bit has a helix angle of about 50°.
9. The method according to item 7 or 8, characterized in that the drill point has a point angle of about 125° to about 135°.
10. The method according to any of items 7 -9, characterized in that the drill point has a point angle of about 130°.
11. The method according to any of items 7 -10, further comprising the step of connecting the mounting portion of the drill bit with the torque monitoring device.
12. The method according to any of items 7 -11, characterized in that the step of quantifying the density of the material based upon the measured torque comprises comparing the measured torque to predetermined torque values of classified bone qualities.
13. A twist drill for assessing bone quality according to the method of any of items 7-12.
14. A tap for assessing bone quality during tapping, comprising:
   a shank having opposed ends;
   a mounting portion formed in one end, said mounting portion being adapted to connect with a torque monitoring device;
   a cutting portion disposed on the other end of said shank, said cutting portion having at least one helical thread formed thereon; and
   a medial, non-cutting portion disposed between said cutting portion and said mounting portion, said medial portion having a diameter less than a diameter of said cutting portion so as to minimize friction forces and enable faster advancement into the bone during tapping.
15. The tap according to item 14, characterized in that the cutting portion has a helix angle of about 45°.
16. The tap according to item 14 or 15, characterized in that the tap has a tapping length of approximately 10 mm.
17. The tap according to any of items 14-16, further comprising a non-cutting, steering portion located at an end of said shank.
18. The tap according to any of items 14-17, characterized in that the cutting portion is comprised of approximately the first four threads.
19. A method of assessing bone quality during tapping, comprising the steps of:
   providing a tap, the tap including a shank having opposed ends, a mounting portion formed in one end of the shank and being adapted to connect with a torque monitoring device, a cutting portion disposed on the other end of said shank, said cutting portion having at least one helical thread formed thereon, and a medial, non-cutting portion disposed between said cutting portion and said mounting portion, said medial portion having a diameter less than a diameter of said cutting portion so as to minimize friction forces and enable faster advancement into the bone during tapping;
   inserting a first few threads of said cutting portion into a predrilled pilot bore of said bone;
   incrementally tapping said cutting portion into the hole and monitoring the torque produced by each turn; and
   quantifying the density of the bone based upon the measured torque to determine the quality of the bone for implantation.
20. The method according to item 19, characterized in that the step of incrementally tapping the cutting portion into the hole comprises advancing the cutting portion of about 1mm with each turn.
21. The method according to item 19 or 20, further comprising the step of connecting the mounting portion of the drill bit with the torque monitoring device.
22. The method according to any of items 19-21, characterized in that the step of quantifying the density of the material based upon the measured torque comprises comparing the measured torque to predetermined torque values of classified bone qualities.
23. The method according to any of items 19-22, characterized in that the cutting portion has a helix angle of about 45°.
24. The method according to any of items 19-23, characterized in that the tap has a tapping length of approximately 10 mm.
25. The method according to any of items 19-24, further comprising a non-cutting, steering portion located at an end of said shank.
26. The method according to any of items 19-25, characterized in that approximately the first four threads of said cutting portion are tapped into the hole.
27. A tap for accessing bone quality during tapping according to the method of any of items 19-26.

## Claims

1. A tap (30) for assessing bone quality during tapping, comprising:
a shank (32) having opposed ends;
a mounting portion (28) formed in one end, said mounting portion being adapted to connect with a torque monitoring device;
a cutting portion (36) disposed on the other end of said shank (32), said cutting portion (36) having at least one helical thread (34) formed thereon; and
a medial, non-cutting portion (38) disposed between said cutting portion (36) and said mounting portion (28), said medial portion (38) having a diameter less than a diameter of said cutting portion so as to minimize friction forces and enable faster advancement into the bone during tapping;
**characterized in that** the cutting portion (36) has a helix angle (β) of about 45°.

2. The tap (30) according to claim 1, **characterized in that** the tap (30) has a tapping length of approximately 10 mm.

3. The tap (30) according to any of claim 1 or 2, further comprising a non-cutting, steering portion (50) located at an end of said shank (32).

4. The tap (30) according to any of claims 1-3, **characterized in that** the cutting portion (36) is comprised of approximately the first four threads.
